# EUROPÄISCHE PATENTANMELDUNG

(11) **EP 2 074 958 A2**
(43) Veröffentlichungstag der Anmeldung: **01.07.2009**
(21) Anmeldenummer: 08020988.5
(22) Anmeldetag: 03.12.2008
(51) Int. Cl.: A61B 17/86, A61B 5/053, A61N 1/05, A61N 1/20, A61N 1/32

(54) **Kontakvorrichtung für die Osteosynthese**

(30) Priorität: 28.12.2007 DE 102007063027
(71) Anmelder: Neue Magnetodyn GmbH, 80333 München (DE)
(72) Erfinder: Kraus, Werner, 80539 München (DE); Stephan, Heribert, 80689 München (DE)
(74) Vertreter: Schumacher & Willsau

(57) **Zusammenfassung**

Die Erfindung betrifft eine Kontaktvorrichtung (10) für eine Knochenschraube (14), wobei in einem implantierten Endzustand der Anordnung aus Knochenschraube (14) und Kontaktvorrichtung (10) ein erster Teil (16) der Kontaktvorrichtung (10) außerhalb eines Hohlraums (12) und ein zweiter Teil (18) innerhalb des Hohlraums (12) der Knochenschraube (14) angeordnet ist, die Teile (16, 18) Gegengegenelektroden bilden und der zweite Teil (18) die Knochenschraube (14) elektrisch kontaktiert, während der erste Teil (16) gegen die Knochenschraube (14) isoliert ist, wobei die Kontaktvorrichtung (10) einen Hohlraum (20, 22) mit einer Spannungsquelle (24, 26) aufweist, deren Pole den ersten Teil (16) und den zweiten Teil (18) jeweils direkt oder indirekt elektrisch kontaktieren.

Um eine solche Kontaktvorrichtung insbesondere im Hinblick auf das für elektrische Komponenten zur Verfügung stehende Volumen, zu verbessern, ist vorgesehen, dass der erste Teil (16) der Kontaktvorrichtung (10) in einem implantierten Endzustand der Anordnung aus Knochenschraube (14) und Kontaktvorrichtung (10) proximal angeordnet ist.

## Beschreibung

Die Erfindung betrifft eine Kontaktvorrichtung zum elektrischen Kontaktieren einer einen Hohlraum aufweisenden, zumindest teilweise elektrisch leitfähigen Knochenschraube, wobei in einem implantierten Endzustand der Anordnung aus Knochenschraube und Kontaktvorrichtung ein erster Teil der Kontaktvorrichtung außerhalb des Hohlraums und ein zweiter Teil der Kontaktvorrichtung innerhalb des Hohlraums der Knochenschraube angeordnet ist, der erste Teil und der zweite Teil der Kontaktvorrichtung zumindest teilweise Gegengegenelektroden bilden und der zweite Teil der Kontaktvorrichtung die Knochenschraube elektrisch kontaktiert, während der erste Teil der Kontaktvorrichtung gegen die Knochenschraube isoliert ist, so dass eine äußere Oberfläche der Knochenschraube zumindest teilweise als Gegenelektrode zu der durch den ersten Teil der Kontaktvorrichtung gebildeten Elektrode wirkt, wobei die Kontaktvorrichtung einen Hohlraum aufweist und in dem Hohlraum mindestens eine Spannungsquelle angeordnet ist, deren Pole den ersten Teil und den zweiten Teil der Kontaktvorrichtung jeweils direkt oder indirekt elektrisch kontaktieren.

Derartige Kontaktvorrichtungen sind auf dem Gebiet der Osteosynthese bekannt. Die Osteosynthese dient der belastungsstabilen Fixation der Fragmente eines gebrochenen oder kranken Knochens in seiner unverletzten, natürlichen Form durch implantierte Schrauben, Stützplatten, Drähte, Knochenmarknägel und dergleichen, die im Allgemeinen aus nicht rostenden Stahl- oder Titanlegierungen gefertigt sind. Diese Osteosynthesemittel ermöglichen die rasche Mobilisierung des Patienten bei gleichzeitiger Ruhigstellung des lädierten Knochens, die eine unerlässliche Voraussetzung für seine Heilung ist.

Problematisch an der starren Fixierung durch die vergleichsweise unelastischen, gewebeverdrängenden Stützimplantate ist jedoch die Behinderung der biologischen Erholung vor allem durch den Verlust von Blutgefäßen und Nerven. Außerdem leidet mit zunehmender Implantationsdauer die biomechanische Qualität der Stützstruktur durch den partiellen Entzug ihrer Funktion. Mit dem Verlust an biologischer Kontrolle aber wächst die Gefahr der Infektion durch resistente Bakterien (MRSA = Multiresistenter Staphylococcus Aureus). Es wurde gezeigt, dass diese die Oberfläche von Metallimplantaten in Form eines adhärenten Biofilmes besiedeln können und mit einer Schleimhülle aus Polysacchariden dem Angriff von Antibiotika widerstehen.

Diesen Problemen kann im Rahmen der orthopädischen Chirurgie durch die Elektro-Osteotherapie begegnet werden, beispielsweise unter Verwendung der eingangs genannten gattungsgemäßen Kontaktvorrichtung, wie beispielsweise in der US 6,778,861 B1 beschrieben. Bei der dort dargestellten magnetisch induzierten Elektro-Osteotherapie werden in Osteosythesemitteln elektrische Wechselpotentiale niedriger Frequenz dadurch induziert, dass ein betroffener Körperteil einem magnetischen Wechselfeld ausgesetzt wird. Seit langem wurde in zahlreichen klinischen Anwendungen der verfahrensgemäßen Technik bei chronisch therapieresistenten, meist infizierten Knochendefekten, Zysten und Tumormetastasen sowie in kliniknahen experimentellen Studien gezeigt, dass mit der Verwendung der Osteosynthese-Implantate als Quellen extrem niederfrequenter sinusförmiger elektrischer Wechselpotentiale in der dem Stützmetall anliegenden Knochenregion ein optimaler Heilungseffekt erzielt wird.

Die Technik der Übertragung funktioniert nach dem Transformatorprinzip: Die verletzte oder kranke Körperregion wird von einem extrem niederfrequenten sinusförmig verlaufenden Magnetfeld mit einer Frequenz von ca. 1 bis 100 Hz - vorzugsweise von 4 bis 20 Hz - und einer magnetischen Flussdichte von 0,5 bis 5 mT (5 bis 50 Gauß) durchflutet, das durch einen Funktionsstromgenerator in einer oder mehreren
- primären - äußeren Stromspulen erzeugt wird, in die das mit den Osteosynthesemitteln versehene Körperteil eingebracht wird. Diese extrem niederfrequenten elektromagnetischen Felder durchdringen weitgehend verlustfrei das Gewebe, einschließlich eventuelle Kleidung und einen Gipsverband, sowie die unmagnetischen (austenitischen) Stützmetalle der Osteosynthese. Im elektrischen Kontakt mit diesen wird eine - sekundäre - Spulenanordnung, der so genannte Übertrager, implantiert. Die in dem Übertrager induzierten Elektropotentiale werden so im Bereich der Knochenläsion sowie allgemein in dem an die Osteosynthesemittel angrenzenden Gewebe zur Wirkung gebracht.

Mit dieser Technik der induktiven Übertragung therapeutisch wirksamer Elektropotentiale auf die Bestandteile der Osteosynthese wird die Infektionsgefahr durch percutane Stromleitungen vermieden, und es können die Behandlungsparameter elektrische Spannung, Frequenz, Intensität, Signalform und die Behandlungszeit mit der indikationsspezifischen Programmierung eines Funktionsstrom-Generators des induzierenden Magnetfeldes bestimmt werden.

Neben der Elektro-Osteosynthese auf der Grundlage der magnetischen Induktion wurde auch bereits vorgeschlagen, die Energiequelle in dem Implantat anzuordnen, so dass für die Erzeugung der heilungsfördernden elektrischen Felder keine aufwändigen externen Apparaturen erforderlich sind und die elektrischen Felder im Idealfall permanent vorliegen, das heißt nicht nur dann, wenn sich der Patient im externen Magnetfeld befindet. An diesen Lösungen ist jedoch problematisch, dass für die Energiequelle ein vergrößertes Volumen in dem Implantat zur Verfügung gestellt werden muss, welches aufgrund der medizinischen Randbedingungen naturgemäß nicht ohne weiteres zur Verfügung steht. Betrachtet man etwa die Fig. 4 der US 6,778,861 B1, so ist das Volumen für die Energiequelle zumindest durch die Länge und den Durchmesser des in die Knochenschraube einführten Einsatzes begrenzt.

Der Erfindung liegt die Aufgabe zugrunde, eine gattungsgemäße Kontaktvorrichtung dahingehend weiterzubilden, dass für die Unterbringung elektrischer Komponenten ein vergrößertes Volumen zur Verfügung steht. Außerdem sollen die Handhabbarkeit und die flexible Verwendbarkeit der Kontaktvorrichtung während der Operation, die biologische Wirkung, die therapeutische Wirksamkeit und die Wirtschaftlichkeit verbessert werden.

Diese Aufgabe wird mit den Merkmalen des unabhängigen Anspruchs gelöst.

Vorteilhafte Ausführungsformen der Erfindung sind in den abhängigen Ansprüchen angegeben.

Die Erfindung baut auf der gattungsgemäßen Kontaktvorrichtung dadurch auf, dass der erste Teil der Kontaktvorrichtung in einem implantierten Endzustand der Anordnung aus Knochenschraube und Kontaktvorrichtung proximal angeordnet ist. Durch diese Anordnung des ersten Teils der Kontaktvorrichtung, welcher außerhalb der Knochenschraube angeordnet ist, steht innerhalb der Kontaktvorrichtung ein vergrößertes Volumen zur Verfügung. Die Kontaktvorrichtung kann in einfacher Weise während der Operation in die bereits implantierte Knochenschraube eingesetzt werden, ohne dass hierfür besondere vorbereitende Maßnahmen erforderlich wären, da insbesondere die in Figur 4 der US 6,778,861 B1 vorhandene Spitze am distalen Ende der Anordnung entfällt und insofern auch kein Vorbohren oder irgendwelche Maßnahmen zur ausreichenden elektrischen Kontaktierung dieser nun nicht mehr vorhandenen Spitze der Kontaktvorrichtung ergriffen werden müssen. Speziell kann praktisch jede herkömmliche kanülierte Schraube mit der Kontaktvorrichtung ausgestattet werden. Der Operateur muss erst nach dem Einsetzen der Knochenschraube entscheiden, ob er diese so belassen oder mit der Kontaktvorrichtung ausstatten möchte. Dies hat insbesondere auch dann Relevanz, wenn mehrere Knochenschrauben zum Fixieren einer Knochenplatte verwendet werden, die dann Teil der elektrifizierten Komponenten wird. Eine oder mehrere Knochenschrauben können dann mit der erfindungsgemäßen Kontaktvorrichtung ausgestattet werden, während die anderen Knochenschrauben ohne Kontaktvorrichtung bleiben können. Jedenfalls muss erst nach vollständig implantierter Anordnung aus Knochenplatte und Knochenschraube entschieden werden, welche Schrauben mit der Kontaktvorrichtung bestückt werden. Durch die erfindungsgemäße Anordnung wird vorzugsweise die gesamte Knochenschraube zur Kathode, während der außerhalb der Knochenschraube liegende erste Teil der Kontaktvorrichtung zumindest teilweise die Anode bildet. Auf diese Weise wird die magnetisch induzierte Osteogenese auf den Stabilisierungsbereich der Knochenschraube, das heißt den Schraubenschaft, konzentriert, da die Osteogenese von der Polarität der jeweiligen Elektroden abhängt, nämlich an der Kathode begünstigt und an der Anode behindert wird. Folglich wird die Knochenbildung in der Umgebung des proximalen Teils der Kontaktvorrichtung behindert, vermieden, und/oder es wird eine Osteolyse bewirkt, während im Bereich der Fraktur die Knochenbildung in erwünschter Weise verstärkt wird. Insbesondere wird hierdurch die Explantation der Knochenschraube vereinfacht, denn die Kontaktvorrichtung kann zum Zwecke der Explantation einfach abgenommen werden, ohne dass dies durch Knochengewebe behindert würde.

Nützlicherweise ist vorgesehen, dass die Kontaktvorrichtung in die Knochenschraube einschiebbar ist. Auf der Grundlage dieser Ausführungsform lässt sich die erfindungsgemäße Kontaktvorrichtung praktisch in jede Knochenschraube einschieben, da an der Innenwand der Knochenschraube keine besonderen Mittel zur Aufnahme der Kontaktvorrichtung vorgesehen sein müssen.

In diesem Zusammenhang ist es von besonderem Vorteil, dass der zweite Teil der Kontaktvorrichtung die Knochenschraube über mindestens einen Federkontakt kontaktiert. Ein oder mehrere Federkontakte stehen nützlicherweise beim Einschieben der Kontaktvorrichtung mit dieser in Verbindung und stützen sich dann ab einer bestimmten Einschubposition und insbesondere dann auch im Endzustand der Anordnung aus Knochenschraube und Kontaktvorrichtung an der Innenwand der hohlen Knochenschraube ab. Die Kontaktvorrichtung kann von vorn herein mit einem oder mehreren solcher Federkontakte ausgestattet sein. Es ist aber auch denkbar, dass der oder die Federkontakte gleichsam als Adapter zwischen der Kontaktvorrichtung und der Knochenschraube wirken, so dass baugleiche Kontaktvorrichtungen durch die Vermittlung unterschiedlicher Federkontakte mit verschiedenartigen Knochenschrauben zusammenwirken können.

Gemäß einer weiteren Ausführungsform der Erfindung ist vorgesehen, dass die Kontaktvorrichtung in die Knochenschraube einschraubbar ist. In diesem Fall muss die Kontaktvorrichtung ein Außengewinde aufweisen, während in dem Hohlraum der Knochenschraube ein Innengewinde vorgesehen ist.

Es ist von besonderem Vorteil, dass die mindestens eine Spannungsquelle einen Akkumulator umfasst. Für einen solchen Akkumulator, beispielsweise einen Lithium-Ionen-Akkumulator, steht nun insbesondere innerhalb des außerhalb der Knochenschraube angeordneten ersten Teils der Kontaktvorrichtung ein ausreichendes Volumen zur Verfügung.

Ebenfalls kann vorgesehen sein, dass die mindestens eine Spannungsquelle einen Kondensator umfasst. Beispielsweise kann ein sogenannter Supercap zur Energiespeicherung vorgesehen sein.

Weiter ist von Vorteil, dass die mindestens eine Spannungsquelle eine Spule umfasst.

Dann ist es von besonderem Vorteil, dass die Spule zu dem Akkumulator und/oder dem Kondensator parallel geschaltet ist. Auch wenn ein Akkumulator beziehungsweise ein Kondensator als Energiequellen ohne eine Spule auskommen und umgekehrt zur Elektrifizierung der Knochenschraube an sich eine Spule zur Aufnahme der Energie eines externen Magnetfeldes ausreichend ist, kann doch eine Kombination von Spule und Akkumulator und/oder Kondensator besonders nützlich sein; hierdurch lässt sich nämlich durch magnetische Induktion die Spule zum Aufladen des oder der Energiespeicher verwenden, so dass die für die Elektrifizierung der Vorrichtung benötigte Energie zwar durch ein externes Magnetfeld geliefert wird, aber auch in Abwesenheit eines externen Magnetfeldes zur Verfügung steht. Auf dieser Grundlage kann es beispielsweise ausreichend sein, wenn ein Implantat einmal täglich für kurze Zeit einem externen magnetischen Wechselfeld ausgesetzt wird, durch das der oder die Energiespeicher geladen wird/werden und die gespeicherte Energie dann für den verbleibenden Zeitraum des Tages zur Verfügung steht.

Es kann von weiterem Vorteil sein, dass in dem Hohlraum der Kontaktvorrichtung ein Funktionsgenerator angeordnet ist. Ein solcher erzeugt vorzugsweise sinusförmige oder ähnliche Wechselspannungen. Wird die Energie durch einen Akkumulator innerhalb der Kontaktvorrichtung geliefert, so steht zunächst eine Gleichspannung zur Verfügung, die grundsätzlich dazu geeignet ist, eine Knochenschraube in der geeigneten Weise zu elektrifizieren. Es kann aber erwünscht sein, eine elektrische Wechselspannung zur Verfügung zu stellen, um auf diese Weise vergleichbare elektrische Bedingungen zu erzeugen, wie sie durch die bekannte Wechselfeldtherapie mit externem Magnetfeld und interner Übertragerspule bekannt sind. Im Zusammenhang mit derartigen Wechselspannungen ist nützlicherweise aber darauf zu achten, dass diese an dem äußeren Teil der Kontaktvorrichtung zumindest vorwiegend positive Polarität erzeugen, während der Schraubenschaft zumindest vorwiegend auf negativem Potential liegt, da auf diese Weise das Knochenwachstum im lädierten Bereich des Knochens gefördert wird und ein unnötiges Einwachsen des äußeren Teils der Kontaktvorrichtung behindert beziehungsweise vermieden wird. Die Bereitstellung dieser Potentiale kann durch die Parallelschaltung einer Diode und gegebenenfalls eines Glättungskondensators zu der durch den Funktionsgenerator gebildeten Spannungsquelle erreicht werden.

Von besonderem Vorteil kann sein, dass in dem Hohlraum der Kontaktvorrichtung eine Messschaltung und eine Schaltung zur Hochfrequenzidentifizierung zum Erfassen und Übertragen von Signalen angeordnet ist, wobei die Signale insbesondere physiologisch relevanten Parametern entsprechen. Durch die Messvorrichtung können beispielsweise eine vom Gewebezustand abhängige Impedanz oder ein pH-Wert gemessen werden. Ebenfalls kann kontinuierlich die anliegende elektrische Spannung überwacht werden. Die entsprechenden Signale können dann über die Schaltung zur Hochfrequenzidentifizierung (RFID) an eine externe Auswerteeinrichtung übertragen werden.

Es kann von Vorteil sein, dass die Kontaktvorrichtung zur Einnahme eines implantierten Endzustands der Anordnung aus Knochenschraube und Kontaktvorrichtung ein Schnapp- oder Rastverbindung mit der Knochenschraube eingeht. Der Operateur hat damit auch unter schwierigen Bedingungen einen weiteren Anhaltspunkt zur korrekten Anordnung der Kontaktvorrichtung innerhalb der Knochenschraube. Die Schnapp- oder Rastverbindung kann weiterhin eine unbeabsichtigte spätere Deplazierung der Kontaktvorrichtung bezüglich der Knochenschraube verhindern.

Die Erfindung betrifft weiterhin eine Osteosyntheseeinrichtung mit einer erfindungsgemäßen Knochenschraube.

Die Erfindung wird nun mit Bezug auf die begleitenden Zeichnungen anhand bevorzugter Ausführungsformen beispielhaft erläutert.

### Es zeigen:

Figur 1 einen in axiale Richtung geführten Schnitt durch eine erste Ausführungsform einer Anordnung aus Knochenschraube und Kontaktvorrichtung und
Figur 2 einen in axiale Richtung geführten Schnitt durch eine zweite Ausführungsform einer Anordnung aus Knochenschraube und Kontaktvorrichtung.

Bei der nachfolgenden Beschreibung der bevorzugten Ausführungsformen der vorliegenden Erfindung bezeichnen gleiche Bezugszeichen gleiche oder vergleichbare Komponenten.

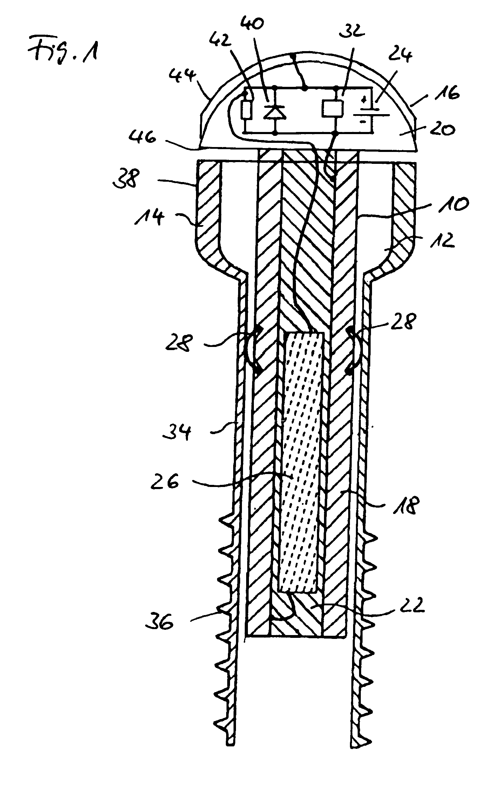

Figur 1 zeigt einen in axiale Richtung geführten Schnitt durch eine erste Ausführungsform einer Anordnung aus Knochenschraube und Kontaktvorrichtung. Es sind eine Knochenschraube 14 und eine in einen Hohlraum 12 der Knochenschraube 14 eingesetzte Kontaktvorrichtung 10 dargestellt.

Die Knochenschraube 14 hat einen Schraubenschaft 34 und einen an dem Schraubenschaft 34 vorgesehenen Gewindeabschnitt 36. Am proximalen Ende des Schraubenschaftes 34 schließt ein Schraubenkopf 38 an, der vorzugsweise mit einer Einrichtung ausgestattet ist, in die ein Werkzeug für das Einschrauben der Knochenschraube 14 eingreifen kann. Beispielsweise wird der Hohlraum 12 der Knochenschraube 14 im Bereich des Schraubenkopfes 38 durch einen Innensechskant oder vergleichbare Form- und/oder Kraftschlusseinrichtungen begrenzt.

Die Kontaktvorrichtung 10 umfasst einen proximal angeordneten kappenförmigen ersten Teil 16 und einen distal angeordneten länglichen zweiten Teil 18, wobei der distale zweite Teil 18 in den Hohlraum 12 der Knochenschraube 14 hineinragt. Zur mechanischen und elektrischen Kontaktierung der Kontaktvorrichtung 10 mit der Knochenschraube 14 sind elektrisch leitende Federkontakte 28 vorgesehen. Zur Verbesserung der mechanischen und elektrischen Kontaktierung können, anders als hier dargestellt, auch eine Vielzahl von Federkontakten an verschiedenen axialen Koordinaten vorgesehen sein. Der kappenförmige erste Teil 16 der Kontaktvorrichtung 10 enthält einen Hohlraum 20, in dem verschiedene elektrische Bauteile angeordnet sind, im vorliegenden Ausführungsbeispiel ein Akkumulator 24, eine Schaltung 32 zur Hochfrequenzidentifizierung 32 (RFID), eine Diode 40 und ein ohmscher Widerstand 42. Der Bereitstellung elektrischer Energie dient der Akkumulator 24, dessen Kathode mit dem länglichen zweiten Teil 18 der Kontaktvorrichtung 10 elektrisch kontaktiert ist und dessen Anode eine elektrisch leitfähige äußere Schale 44 des kappenförmigen ersten Teils 16 der Kontaktvorrichtung 10 kontaktiert. Durch die elektrische Kontaktierung des länglichen zweiten Teils 18 der Kontaktvorrichtung 10 mit der Knochenschraube 14 wird diese zur Kathode, während die äußere elektrisch leitfähige Schale 44 des kappenförmigen ersten Teils 16 der Kontaktvorrichtung 10 die Anode bildet.

Ein Kurzschluss zwischen diesen beiden Gegenelektroden ist zu vermeiden, wobei zu diesem Zweck vorgesehen sein kann, dass der kappenförmige erste Teil 16 der Kontaktvorrichtung 10 einen elektrisch isolierenden Bereich 46 aufweist, der die distale Begrenzung des kappenförmigen ersten Teils 16 der Kontaktvorrichtung 10 bildet. Der isolierende Bereich 46 kann beispielsweise durch eine DLC-Schicht realisiert sein (DLC = diamond-like carbon). Alternativ oder zusätzlich können weitere isolierende Einrichtungen zwischen dem kappenförmigen ersten Teil 16 der Kontaktvorrichtung 10 und der Knochenschraube 14 vorgesehen sein. Diese isolierenden Einrichtungen können fest mit der Knochenschraube 14, fest mit dem kappenförmigen ersten Teil 16 der Kontaktvorrichtung 10 oder auch als separates Bauteil ausgeführt sein.

Der Endzustand der implantierten Anordnung aus Kontaktvorrichtung 10 und Knochenschraube 14 kann fest definiert sein, etwa durch einen Anschlag, der beispielsweise durch einen Kontakt zwischen dem isolierenden Teil 46 des kappenförmigen ersten Teils 16 der Kontaktvorrichtung gebildet ist, oder der auch durch eine Schnapp- oder Rastverbindung realisiert sein kann. Ebenfalls kann der Endzustand der implantierten Anordnung aus Kontaktvorrichtung 10 und Knochenschraube 14 variabel sein, so dass der Operateur während der Operation entscheiden kann, die Kontaktvorrichtung 10 weiter oder weniger weit in die Knochenschraube einzuschieben.

Nützlicherweise sind die elektrischen Komponenten innerhalb des Hohlraums 20 des kappenförmigen ersten Teils 16 der Kontaktvorrichtung 10 in eine elektrisch isolierende Masse eingebettet, etwa ein gewebeverträgliches Gießharz. Gleiches gilt für den Hohlraum 22, welcher sich innerhalb des länglichen zweiten Teils 18 der Kontaktvorrichtung 10 befindet. Im vorliegenden Ausführungsbeispiel ist innerhalb dieses Hohlraums 22 eine Spule 26, die vorzugsweise einen weichmagnetischen Kern aufweist, angeordnet. Diese Spule dient als Energielieferant zum Aufladen des Akkumulators 24, nämlich dann, wenn die Anordnung aus Knochenschraube 14 und Kontaktvorrichtung 10 in einem äußeren magnetischen Wechselfeld liegt. Zu diesem Zweck ist die Spule 26 elektrisch parallel zu dem Akkumulator 24 geschaltet, das heißt ein Pol der Spule steht mit der Anode des Akkumulators 24 in Verbindung, während der andere Pol der Spule mit der Kathode des Akkumulators 24 verbunden ist, hier unter Zwischenschaltung des elektrisch leitfähigen länglichen zweiten Teils 18 der Kontaktvorrichtung 10. Auch eine unmittelbare Kontaktierung der elektrischen Komponenten ist möglich.

Die weiteren in dem ersten Teil 16 der Kontaktvorrichtung 10 angeordneten elektrischen Komponenten dienen verschiedenen Zwecken. Die Anordnung des RFID 32 in dem kappenförmigen ersten Teil 16 der Kontaktvorrichtung 10 ist schematisch zu verstehen. Wesentlich ist, dass dieser RFID 32 Signale an eine externe Auswerteeinrichtung übergeben kann, die auf Ausgangssignalen von internen Messschaltungen beruhen. Die Diode 40 dient der Gleichrichtung der Spannung, nämlich dann, wenn diese direkt durch die Spule 26 geliefert wird. Der ohmsche Widerstand 42 steht stellvertretend für verschiedene weitere elektrische Komponenten, über die die Spannungsverläufe modifiziert werden können.

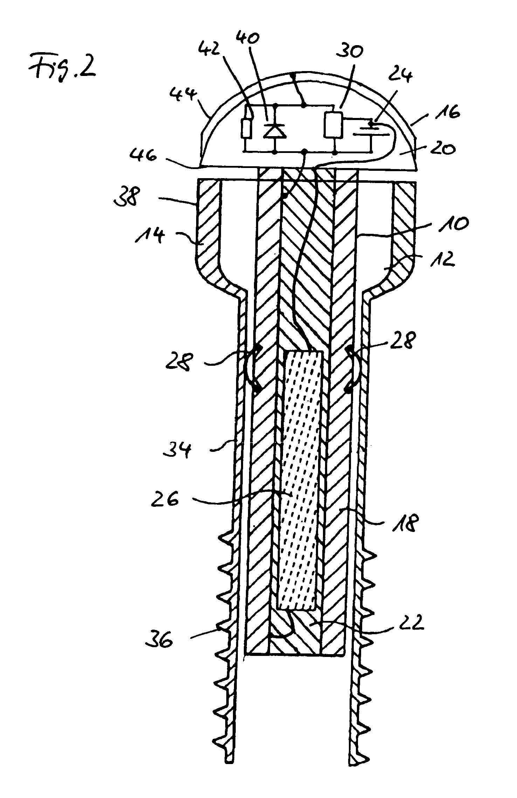

Figur 2 zeigt einen in axiale Richtung geführten Schnitt durch eine zweite Ausführungsform einer Anordnung aus Knochenschraube und Kontaktvorrichtung. Hier kommt ein Funktionsgenerator 30 zum Einsatz, der über seine Eingangsklemmen von dem Akkumulator 24 mit Energie versorgt wird, während seine Ausgangsklemmen die beiden Teile 16, 18 der Kontaktvorrichtung 10 kontaktieren. Die Diode kann wiederum dafür sorgen, dass der proximale kappenförmige erste Teil 16 vorwiegend positives Potential hat, während die Knochenschraube 14 eine vorwiegend kathodische Komponente erhält.

Ebenso wie im Zusammenhang mit Figur 1 erläutert, kann auch bei der hier dargestellten Ausführungsform mit Funktionsgenerator 30 ein der Telemetrie dienender RFID vorgesehen sein.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### Bezugszeichenliste

- 10: Kontaktvorrichtung
- 12: Hohlraum
- 14: Knochenschraube
- 16: erster Teil
- 18: zweiter Teil
- 20: Hohlraum
- 22: Hohlraum
- 24: Akkumulator
- 26: Spule
- 28: Federkontakt
- 30: Funktionsgenerator
- 32: Schaltung
- 34: Schraubenschaft
- 36: Gewinde
- 38: Schraubenkopf
- 40: Diode
- 42: ohmscher Widerstand
- 44: leitfähige Schale
- 46: isolierendes Gehäuse

## Patentansprüche

1. Kontaktvorrichtung (10) zum elektrischen Kontaktieren einer einen Hohlraum (12) aufweisenden, zumindest teilweise elektrisch leitfähigen Knochenschraube (14), wobei in einem implantierten Endzustand der Anordnung aus Knochenschraube (14) und Kontaktvorrichtung (10) ein erster Teil (16) der Kontaktvorrichtung (10) außerhalb des Hohlraums (12) und ein zweiter Teil (18) der Kontaktvorrichtung (10) innerhalb des Hohlraums (12) der Knochenschraube (14) angeordnet ist, der erste Teil (16) und der zweite Teil (18) der Kontaktvorrichtung (10) zumindest teilweise Gegengegenelektroden bilden und der zweite Teil (18) der Kontaktvorrichtung die Knochenschraube (14) elektrisch kontaktiert, während der erste Teil (16) der Kontaktvorrichtung (10) gegen die Knochenschraube (14) isoliert ist, so dass eine äußere Oberfläche der Knochenschraube (14) zumindest teilweise als Gegenelektrode zu der durch den ersten Teil (16) der Kontaktvorrichtung (10) gebildeten Elektrode wirkt, wobei die Kontaktvorrichtung (10) einen Hohlraum (20, 22) aufweist und in dem Hohlraum (20, 22) mindestens eine Spannungsquelle (24, 26) angeordnet ist, deren Pole den ersten Teil (16) und den zweiten Teil (18) der Kontaktvorrichtung (10) jeweils direkt oder indirekt elektrisch kontaktieren, **dadurch gekennzeichnet, dass** der erste Teil (16) der Kontaktvorrichtung (10) in einem implantierten Endzustand der Anordnung aus Knochenschraube (14) und Kontaktvorrichtung (10) proximal angeordnet ist.

2. Kontaktvorrichtung nach Anspruch 1, **dadurch gekennzeichnet, dass** die Kontaktvorrichtung (10) in die Knochenschraube (14) einschiebbar ist.

3. Kontaktvorrichtung nach Anspruch 1 oder 2, **dadurch gekennzeichnet, dass** der zweite Teil (18) der Kontaktvorrichtung (10) die Knochenschraube (14) über mindestens einen Federkontakt (28) kontaktiert.

4. Kontaktvorrichtung nach einem der vorangehenden Ansprüche, **dadurch gekennzeichnet, dass** die Kontaktvorrichtung (10) in die Knochenschraube (14) einschraubbar ist.

5. Kontaktvorrichtung nach einem der vorangehenden Ansprüche, **dadurch gekennzeichnet, dass** die mindestens eine Spannungsquelle einen Akkumulator (24) umfasst.

6. Kontaktvorrichtung nach einem der vorangehenden Ansprüche, **dadurch gekennzeichnet, dass** die mindestens eine Spannungsquelle einen Kondensator umfasst.

7. Kontaktvorrichtung nach einem der vorangehenden Ansprüche, **dadurch gekennzeichnet, dass** die mindestens eine Spannungsquelle eine Spule (26) umfasst.

8. Kontaktvorrichtung nach Anspruch 7, **dadurch gekennzeichnet, dass** die Spule (26) zu dem Akkumulator (24) und/oder dem Kondensator parallel geschaltet ist.

9. Kontaktvorrichtung nach einem der vorangehenden Ansprüche, **dadurch gekennzeichnet, dass** in dem Hohlraum (20) der Kontaktvorrichtung (10) ein Funktionsgenerator (30) angeordnet ist.

10. Kontaktvorrichtung nach einem der vorangehenden Ansprüche, **dadurch gekennzeichnet, dass** in dem Hohlraum (20) der Kontaktvorrichtung (10) eine Messschaltung und eine Schaltung (32) zur Hochfrequenzidentifizierung zum Erfassen und Übertragen Signalen angeordnet ist, wobei die Signale insbesondere physiologisch relevanten Parametern entsprechen.

11. Kontaktvorrichtung nach einem der vorangehenden Ansprüche, **dadurch gekennzeichnet, dass** die Kontaktvorrichtung (10) zur Einnahme eines implantierten Endzustands der Anordnung aus Knochenschraube (14) und Kontaktvorrichtung (10) ein Schnapp- oder Rastverbindung mit der Knochenschraube (14) eingeht.

12. Osteosyntheseeinrichtung mit einer Knochenschraube (14) und mit einer Kontaktvorrichtung (10) nach einem der vorangehenden Ansprüche.
